# EUROPÄISCHE PATENTSCHRIFT

(11) **EP 0 593 879 B1**
(45) Veröffentlichungstag und Bekanntmachung des Hinweises auf die Patenterteilung: **15.10.1997**
(21) Anmeldenummer: 93113621.2
(22) Anmeldetag: 26.08.1993
(51) Int. Cl.: A61M 3/02

(54) **Behandlungseinheit zur Colon-Hydro-Therapie**
Treatment apparatus for colon hydrotherapy
Dispositif de traitement pour l'hydrothérapie du colon

(30) Priorität: 22.09.1992 DE 9212723 U
(43) Veröffentlichungstag der Anmeldung: 27.04.1994
(73) Patentinhaber: KRESS GmbH, D-63768 Hösbach (DE)
(72) Erfinder: Kress, Günter, D-63768 Hösbach (DE)
(74) Vertreter: Pöhner, Wilfried Anton, Dr.

(56) Entgegenhaltungen:
- EP-A- 0 116 986
- WO-A-92/12760
- US-A- 4 704 102
- US-A- 4 850 965
- US-A- 4 913 698

## Beschreibung

Die Erfindung bezieht sich auf eine Behandlungseinheit zur Colon-Hydro-Therapie mit einem Speculum, einem zum Speculum führenden Zulaufschlauch und einem in Gegenrichtung verlaufenden Ablaufschlauch, wobei eine Kupplung sowie eine Absperrung im Zulaufschlauch nahe, jedoch in derartigem Abstand zum Speculum angebracht ist, daß die Zugänglichkeit der Kupplung gewährleistet ist, wenn das Speculum in das Rektum eines Patienten eingebracht ist.

In jüngster Zeit nehmen als Folge falscher Ernährung, mangelnder Bewegung, Streß und anderen Zivilisationseinflüssen die Erkrankungen des Verdauungstraktes zu. Hierzu zählen neben anderen Krankheitsbildern die Obstipation, bei denen die Verweildauer der Ausscheidungsstoffe im Dickdarm wesentlich erhöht ist, so daß die entstehenden Fäulnis- und Gärungsgifte in größeren Mengen resorbiert werden, die zwangsläufig zu einer Auto-Intoxikation des Körpers führen. Zahlreiche Erkrankungen, wie Infektionen, Entzündungen, Rheuma, Polyarthritis, Akne und andere Hauterkrankungen, lassen sich zumindest teilweise auf die Auto-Intoxikation zurückführen. Die Anwendung von Abführmitteln und anderer Medikamente wirken in nachteiliger Weise zerstörend auf die Darmschleimhaut.

Im professionellen Einsatz findet auch zur Vermeidung anderer, für den Patienten subjektiv als negativ empfundener Begleiterscheinungen zur Reinigung und Sanierung des Darmes immer mehr die Colon-Hydro-Therapie Anwendung. Hier wird gefiltertes und temperiertes Wasser bis zum Erreichen der Stelle der Obstipation, die sich durch einen Druckanstieg der Infusion anzeigt, in den Dickdarm infundiert und, durch eventuell mehrmaliges Wechseln zwischen Füllen und sofortigem Ausspülen auch hartnäckige Ablagerungen gelöst und über einen zweiten, dem Ablauf dienenden Schlauch entfernt. Die Colon-Hydro-Therapie ist hygienisch und geruchfrei und wird vom Patienten subjektiv als entspannend und frei von unangenehmen Begleiterscheinungen und Verletzung ästhetischer Gefühle empfunden.
Die Behandlungseinheit besteht aus einem Gerät zur Colon-Hydro-Therapie, das der Steuerung und Versorgung der gesamten Einheit dient. Zu diesem Zwecke weist es einen Anschlußstutzen auf, durch den Warmwasser vorgebbarer Temperatur zur Verfügung gestellt wird und an dem ein zur Weiterleitung an das später noch zu erläuternde Speculum dienender Zulaufschlauch angebracht ist. Für gewisse Formen der Behandlungen ist eine Möglichkeit zur Beimischung von Sauerstoff vorgesehen. Einstellbare Schalter für Druck und Temperatur sind selbstverständlich. Schließlich ist noch ein Rücklaufanschluß vorgesehen, an den ein vom Patienten zurückführender Ablaufschlauch zur Ableitung des gelösten Stuhles angeschlossen ist. Gleichzeitig ist das Gerät an eine Wasserzuleitung, in der Regel an eine Warm-/Kaltwasserzuleitung, angeschlossen, die im letzteren Fall die Einstellung der gewünschten Temperatur über eine Mischbatterie vorzunehmen gestattet.

Ein Anschluß an das Abflußrohr sowie an die elektrische Energieversorgung des Gebäudes vervollständigt das Gerät. Sowohl der Zu- als auch Ablaufschlauch sind an ihren äußeren Enden an das Speculum angeschlossen, das durch eine entfernbare Einführhilfe in das Rektum des Patienten zu Beginn der Behandlung einzubringen ist.
Mit Beendigung der Behandlung werden Zulaufschlauch, Speculum mit Einführhilfe und Ablaufschlauch vom Gerät abgezogen und in ihrer Gesamtheit als Müll beseitigt und für den nächsten Patienten völlig neue Schläuche mit Speculum und Einführhilfe eingesetzt.
Der entscheidende Nachteil ist, daß bei professionellem Einsatz nach einer gewissen Dauer der Verwendung eine erhebliche Menge an Müll anfällt, den es zu beseitigen gilt. Des weiteren bereitet es für den in seinem Durchmesser wesentlich geringeren Zulaufschlauch insbesondere bei entsprechend geringen Eigentemperaturen des Materiales in der Winterzeit Probleme, den Schlauch auf den Stutzen sowohl des Gerätes als auch des Speculums aufzuziehen.

Wie die Druckschrift US 4 704 102 zeigt, ist es bei Kathetern zur desinfizierenden Spülung von Körperhöhlungen bekannt, die Elemente der Behandlungseinheit durch mit Absperrungen versehene Kupplungen zu verbinden. Dabei dienen die Absperrungen zur Regulierung der Flüssigkeitsströme und gestatten den Austausch der Elemente während der Behandlung.

Hiervon ausgehend hat sich die Erfindung die Weiterentwicklung derartiger Behandlungseinheiten zur Colon-Hydro-Therapie zur Aufgabe gemacht.

Ausgehend von einem Gerät gattungsgemäßer Art, wird die Aufgabe erfindungsgemäß dadurch gelöst, daß der Zulaufschlauch das Speculum mit einem Gerät verbindet, das zur Steuerung und Zufuhr von Warmwasser einstellbarer Temperatur und Druck unter eventueller Beimengung von Sauerstoff dient, das Speculum mit einer Einführhilfe zur Positionierung im Rektum versehen ist, der Ablaufschlauch einen gegenüber dem Zulaufschlauch größeren Durchmesser aufweist und beide Schläuche im zentralen Kanal des Speculums enden.

Der Grundgedanke der Erfindung besteht darin, im Zulaufschlauch in der Nähe, jedoch in einem Abstand zum Speculum eine Kupplung sowie eine Absperrung vorzusehen, die im gelösten Zustand der Kupplung den Zulaufschlauch sperrt, hingegen in geöffnetem Zustand den Durchlauf frei gibt.

Der Ort der Anbringung auf dem Zulaufschlauch bestimmt sich nach folgenden Überlegungen:
Einerseits sollte die Menge des angefallenen Mülls, den es zu beseitigen gilt, möglichst gering gehalten werden, so daß eine Anbringung unmittelbar in Nähe des Speculums angezeigt erscheint. Dem steht entgegen, daß eine Zugänglichkeit der Kupplung im eingesetzten Zustand auch bei korpulenten Personen gewährleistet sein muß. In Erfüllung beider Forderungen wird als Kompromiß die Anordnung der Kupplung in der Nähe, jedoch im Abstand zum Speculum als optimal vorgeschlagen. Als weiteres Merkmal kommt hinzu, daß im Bereich der Kupplung eine Absperrung des Schlauches vorhanden ist, die in funktioneller Hinsicht beim Trennen eine Aktivierung erfährt und den Schlauch verschließt. Es ist sichergestellt, daß die im zum Gerät hinweisenden Bereich des Schlauches befindliche Infusionsflüssigkeit zurückgehalten wird.

Die mit der Erfindung erreichbaren Vorteile sind unter mehreren Gesichtspunkten entscheidend:
Ausgangspunkt der Erfindung war die Vermeidung oder doch zumindest die Reduzierung des anfallenden Mülles, die dadurch erreichbar ist, daß neben dem Ablaufschlauch und dem Speculum lediglich ein kurzes, auf dem Speculum befestigtes Teil des Ablaufschlauches ausgetauscht werden muß, im übrigen hingegen weitere Verwendung finden kann. Der Müllanfall wird geringer und es läßt sich aufgrund Materialersparnis eine Kostenreduzierung der auszutauschenden Einwegteile erreichen. Aufgrund der Tatsachen, daß zum einen der Zulaufschlauch ständig an dem der Versorgung dienenden Gerät befestigt bleibt und zum anderen im Einwegbereich der anzuliefernde und auf dem Speculum aufsitzende Teil herstellerseitig bereits aufgebracht ist, ergibt sich eine wesentliche Vereinfachung und Beschleunigung der mit dem Austausch erforderlich werdenden Arbeiten.

In einer Weiterbildung ist vorgesehen, die im Zulaufschlauch befindliche Absperrung in der Kupplung zu integrieren.

Durch entsprechende mechanische Verbindungen läßt sich zusätzlich erreichen, daß bei einem Trennen der Kupplung die Absperrung aktiviert und der Zulaufschlauch demzufolge verschlossen ist. Umgekehrt wird bei einer Verbindung beider Elemente der Zulaufschläuche und deren Zusammenpressen im Inneren der Kupplung die Absperrung gelöst und die Beaufschlagung des Speculums realisiert.

Schließlich ist vorgesehen, den Abstand der Kupplung etwa 25 cm von der Befestigung des Zulaufschlauches am Speculum vorzusehen. Dieser Abstand hat sich im Hinblick auf den wechselnden anatomischen Aufbau und dem Ziel der Einsparung von Material als vorteilhaft erwiesen.

Weitere Einzelheiten, Merkmale und Vorteile der Erfindung lassen sich dem nachfolgenden Beschreibungsteil entnehmen, in dem die Erfindung anhand einer in Explosionsdarstellung widergegebenen Ausführungsform näher erläutert ist.

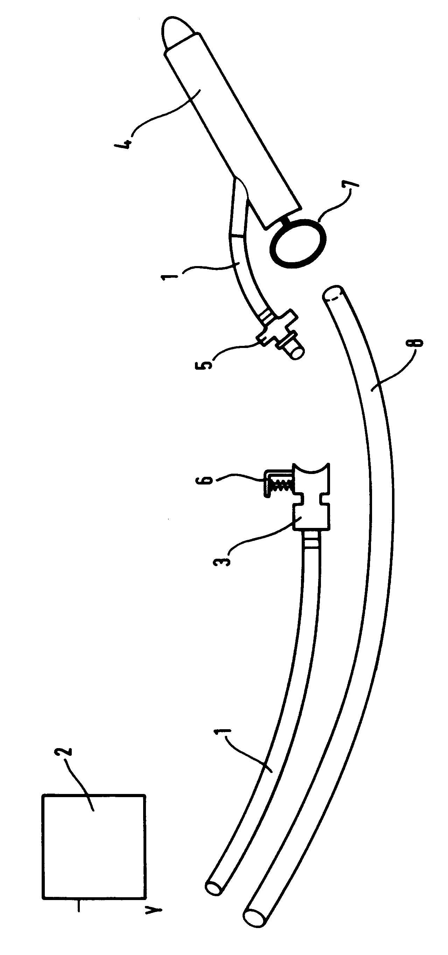

Die in der Zeichnung wiedergegebene Behandlungseinheit ist in demontiertem Zustand dargestellt. In der Mitte der Zeichnung findet sich der Zulaufschlauch (1), der zur Befestigung an dem der Steuerung und Versorgung dienenden Gerät (2) bestimmt ist. Der Schlauch (1) ist außermittig und durch eine Kupplung (3) unterteilt, die in Richtung auf das Speculum (4) zu angeordnet ist. Die beiden Teile des Schlauches (1) werden durch Zusammenwirken von Kupplung (3) und deren Kupplungsgegenstück (5), das mit dem kürzeren Teil des Schlauches in Verbindung steht, verknüpft oder getrennt. Hierzu wird eine Entriegelungstaste (6) betätigt, die gleichzeitig die im Inneren der Kupplung (3) befindliche Absperrung aktiviert, das heißt schließt oder öffnet. Sie stellt sicher, daß bei getrenntem Zulaufschlauch (1) die Infusionsflüssigkeit nicht austreten kann.

Das Speculum (4) ist in jener Form widergegeben, wie sie dem Kunden seitens des Herstellers zur Verfügung gestellt wird. Im Inneren befindet sich folglich die Einführhilfe (7), die nach dem Setzen entfernt wird und anschließend erst die Möglichkeit bietet, den Ablaufschlauch (8) aufzuziehen und im übrigen ebenfalls am Gerät (2) zu befestigen.

Die Funktion wurde bereits eingehend erläutert und entspricht der aus dem Stand der Technik bekannten Behandlungseinheit. Der Vorteil der Erfindung besteht im rascheren Austausch des Einwegmateriales und in der Reduzierung der anfallenden Abfallmenge.

## Patentansprüche

1. Behandlungseinheit zur Colon-Hydro-Therapie mit einem Speculum (4), einem zum Speculum (4) führenden Zulaufschlauch (1) und einem in Gegenrichtung verlaufenden Ablaufschlauch (8), wobei eine Kupplung (3) sowie eine Absperrung im Zulaufschlauch (1) nahe, jedoch in derartigem Abstand zum Speculum (4) angebracht ist, daß die Zugänglichkeit der Kupplung (3) gewährleistet ist, wenn das Speculum (4) in das Rektum eines Patienten eingebracht ist, **dadurch gekennzeichnet**, daß
- die Behandlungseinheit ein Gerät (2) zur Steuerung und Zufuhr von Warmwasser einstellbarer Temperatur und Druck unter eventueller Beimengung von Sauerstoff umfasst,
- der Zulaufschlauch (1) das Speculum (4) mit dem Gerät (2) zur Steuerung und Zufuhr von Warmwasser verbindet,
- das Speculum (4) mit einer Einführhilfe (7) zur Positionierung im Rektum versehen ist,
- der Ablaufschlauch (8) einen gegenüber dem Zulaufschlauch (1) größeren Durchmesser aufweist
- und beide Schläuche (1, 8) im zentralen Kanal des Speculums (4) enden.

2. Behandlungseinheit nach Anspruch 1, **dadurch gekennzeichnet**, daß die Absperrung in der Kupplung (3) integriert ist.

3. Behandlungseinheit nach Anspruch 1 oder 2, **dadurch gekennzeichnet**, daß das Aufschieben und Trennen der Kupplung (3) das Öffnen und Schließen der Absperrung betätigt.

4. Behandlungseinheit nach einem der Ansprüche 1 bis 3, **dadurch gekennzeichnet**, daß der Abstand zwischen Kupplung (3) und Anschlußpunkt des Speculums (4) etwa 25 cm beträgt.

## Claims

1. Treatment unit for colon hydrotherapy having a speculum (4), a feed hose (1) leading to the speculum and a drain hose (8) extending in the opposite direction, where a coupling (3) and a cut-off are attached in proximity to the feed hose (1), but at such a distance in relation to the speculum (4) that ensures accessibility to coupling (3) when speculum (4) is inserted in the rectum of the patient, **wherein**
- said treatment unit encompasses a device (2) to control and supply warm water of a determinable temperature and pressure with the possible addition of oxygen,
- said feed hose (1) combines said speculum (4) with said device (2) for controlling and supplying warm water,
- said speculum (4) is provided with an insertion aid (7) for positioning in the rectum,
- said drain hose (8) has a larger diameter than that of feed hose (1)
- and both hoses (1, 8) end in the central channel of said speculum (4).

2. Treatment unit according to claim 1, **wherein** said cut-off is integrated in coupling (3).

3. Treatment unit according to claim 1 or 2, **wherein** the attachment or separation of said coupling (3) actuates the opening and closing of the cut-off.

4. Treatment unit according to one of claims 1 to 3, **wherein** the distance between coupling (3) and the connection point of speculum (4) is more or less 25 cm.

## Revendications

1. Unité de traitement pour l'hydrothérapie du côlon avec un spéculum (4), un tuyau d'amenée (1) vers le spéculum (4) et un tuyau d'évacuation (8) dont le flux s'effectue en sens inverse, un raccord (3) ainsi qu'un dispositif de fermeture étant fixés dans le tuyau d'amenée (1) à proximité du spéculum (4) tout en maintenant une distance suffisante au spéculum (4) pour que l'accès au raccord (4) soit assuré quand le spéculum (4) est positionné dans le rectum d'un patient, **caractérisée par le fait**
- que l'unité de traitement comprend un appareil (2) pour la commande et l'amenée d'eau chaude de température et de pression réglables éventuellement avec adjonction d'oxygène
- que le tuyau d'amenée (1) relie le spéculum (4) à l'appareil (2) destiné à la commande et à l'amenée de l'eau chaude,
- que le spéculum (4) est muni d'un dispositif facilitant l'introduction dans le rectum (7),
- que le tuyau d'évacuation (8) a un diamètre plus important que le tuyau d'amenée (1)
- et que les deux tuyaux (1, 8) aboutissent dans le canal central du spéculum (4).

2. Unité de traitement pour l'hydrothérapie du côlon selon la revendication 1, **caractérisée en ce que** le dispositif de fermeture est intégré dans le raccord (3).

3. Unité de traitement selon la revendication 1 ou 2, **caractérisée en ce que** l'accouplement et le désaccouplement du raccord (3) déclenchent l'ouverture et la fermeture du dispositif de fermeture.

4. Unité de traitement selon l'une des revendications 1 à 3, **caractérisée par le fait que** la distance entre le raccord (3) et le point de raccord du spéculum (4) est d'environ 25 cm.
